(19) Europäisches Patentamt / European Patent Office / Office européen des brevets

(11) EP 3 300 771 B1

(12) **EUROPÄISCHE PATENTSCHRIFT**

(45) Veröffentlichungstag und Bekanntmachung des Hinweises auf die Patenterteilung:
**17.06.2020 Patentblatt 2020/25**

(51) Int Cl.:
***A61Q 15/00*** *(2006.01)* ***A61K 8/49*** *(2006.01)*

(21) Anmeldenummer: **17196695.5**

(22) Anmeldetag: **21.11.2012**

(54) **VERWENDUNG VON ISOSORBIDCAPRYLATEN/CAPRATEN IN DEODORANTIEN UND ANTIPERSPIRANTIEN**

USE OF ISOSORBIDE CAPRYLATE/CAPRATE IN DEODORANTS AND ANTIPERSPIRANTS

UTILISATION D'ISOSORBIDECAPRYLATES/CAPRATES DANS DES DÉODORANTS ET DES ANTI-TRANSPIRANTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.11.2011 DE 102011119033**

(43) Veröffentlichungstag der Anmeldung:
**04.04.2018 Patentblatt 2018/14**

(62) Dokumentnummer(n) der früheren Anmeldung(en) nach Art. 76 EPÜ:
**12798153.8 / 2 782 645**

(73) Patentinhaber: **Clariant International Ltd**
**4132 Muttenz (CH)**

(72) Erfinder:
- **FRICKE, Tom**
  **65835 Liederbach (DE)**
- **KLUG, Peter**
  **63762 Grossostheim (DE)**
- **CHALUPPA, Carina**
  **65366 Geisenheim (DE)**

(74) Vertreter: **Kampen, Daniela**
**Clariant Produkte (Deutschland) GmbH**
**Patent & License Management Chemicals**
**Industriepark Höchst / G 860**
**65926 Frankfurt am Main (DE)**

(56) Entgegenhaltungen:
**WO-A1-2013/017263** **WO-A2-2010/108738**
**DE-A1-102009 022 444**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 300 771 B1

## Beschreibung

Die vorliegende Erfindung betrifft die Verwendung von Isosorbidcaprylaten/capraten in Antiperspirantien und Deodorantien zur Verbesserung deren Wirkung zur Verringerung von Körpergeruch.

Es sind zahlreiche Aktivstoffe bekannt, die in Antiperspirantien und Deodorantien zur Bekämpfung von Körpergeruch eingesetzt werden können.

Nachteilig an der Verwendung vieler dieser Aktivstoffe ist jedoch, dass ihre Herstellung oftmals aufwändig ist und auf synthetischen Rohstoffen basiert. Für einige dieser Aktivstoffe wie z. B. für Aluminium-haltige Substanzen werden zudem aus toxikologischen Gründen Alternativen gesucht. Darüber hinaus ist die Wirkung der Aktivstoffe gegen Körpergeruch oftmals verbesserungsbedürftig, so dass hohe Einsatzkonzentrationen für eine ausreichende Wirkung notwendig sind. Von Seiten der Konsumenten werden zunehmend Antiperspirantien und

Deodorantien mit langanhaltender Wirkung über 24 Stunden hinaus gefordert.

Es bestand deshalb die Aufgabe, Verbindungen zur Verfügung zu stellen, die die Wirkung der Antiperspirantien und Deodorantien hinsichtlich der Dauer der Verringerung von Körpergeruch verbessern. Sie sollten darüber hinaus auf nachwachsenden Rohstoffen basieren und toxikologisch unbedenklich sein.

Überraschend wurde nun gefunden, dass diese Aufgabe gelöst wird durch Zusammensetzungen enthaltend ein oder mehrere Isosorbidcaprylate/caprate. Gegenstand der Erfindung ist somit die Verwendung einer Zusammensetzung enthaltend ein oder mehrere Isosorbidcaprylate/caprate (Zusammensetzung A) in Antiperspirantien und Deodorantien zur Verbesserung deren Wirkung zur Verringerung von Körpergeruch.

Unter Isosorbidcaprylaten/capraten werden sowohl die reinen Caprylate oder Caprate als auch Mischungen aus Caprylaten und Capraten verstanden.

In einer bevorzugten Ausführungsform der Erfindung handelt es sich bei den Isosorbidcaprylaten/capraten um Mischungen aus Caprylaten und Capraten vorzugsweise im molaren Verhältnis von 1:2 bis 2:1 und besonders bevorzugt im molaren Verhältnis von 2:3 bis 3:2.

In einer weiteren bevorzugten Ausführungsform der Erfindung handelt es sich bei den Isosorbidcaprylaten/capraten um Caprylate mit einem Anteil der Caprate von maximal 5 Mol-%, besonders bevorzugt von maximal 0,5 Mol-% und insbesondere bevorzugt um reine Caprylate, die frei sind von Capraten.

In einer weiteren bevorzugten Ausführungsform der Erfindung handelt es sich bei den Isosorbidcaprylaten/capraten um Caprate mit einem Anteil der Caprylate von maximal 5 Mol-%, besonders bevorzugt von maximal 0,5 Mol-% und insbesondere bevorzugt um reine Caprate, die frei sind von Caprylaten.

Unter Körpergeruch wird vorzugsweise Schweißgeruch verstanden.

In einer weiteren bevorzugten Ausführungsform der Erfindung enthalten die Zusammensetzungen A neben dem einen oder den mehreren Isosorbidcaprylaten/ capraten zusätzlich ein oder mehrere Sorbitancaprylate/caprate.

In einer bevorzugten Ausführungsform der Erfindung handelt es sich bei den Sorbitancaprylaten/capraten um Mischungen aus Caprylaten und Capraten vorzugsweise im molaren Verhältnis von 1:2 bis 2:1 und besonders bevorzugt im molaren Verhältnis von 2:3 bis 3:2.

In einer weiteren bevorzugten Ausführungsform der Erfindung handelt es sich bei den Sorbitancaprylaten/capraten um Caprylate mit einem Anteil der Caprate von maximal 5 Mol-%, besonders bevorzugt von maximal 0,5 Mol-% und insbesondere bevorzugt um reine Caprylate, die frei sind von Capraten.

In einer weiteren bevorzugten Ausführungsform der Erfindung handelt es sich bei den Sorbitancaprylaten/capraten um Caprate mit einem Anteil der Caprylate von maximal 5 Mol-%, besonders bevorzugt von maximal 0,5 Mol-% und insbesondere bevorzugt um reine Caprate, die frei sind von Caprylaten.

Bei den Isosorbidcaprylaten/capraten kann es sich um Isosorbidmonocaprylat/caprat, Isosorbiddicaprylat/caprat oder um beliebige Mischungen daraus handeln. Bei den Sorbitancaprylaten/capraten kann es sich um Mono-, Di-, Tri- oder Tetracaprylat/caprat oder um beliebige Mischungen daraus handeln.

Sorbitancaprylate/caprate und Isosorbidcaprylate/caprate basieren auf nachwachsenden Rohstoffen und sind toxikologisch unbedenklich.

Die Verwendung von Verbindungen wie Isosorbidcaprylaten/capraten und/oder Sorbitancaprylaten/capraten in Zusammensetzungen wie z. B. in kosmetischen, dermatologischen oder pharmazeutischen Zusammensetzungen ist bereits bekannt.

DE 10 2009 022 444 (Clariant) beschreibt flüssige Zusammensetzungen enthaltend Sorbitanmonocaprylat und antimikrobielle Wirkstoffe wie z. B. spezielle organische Säuren und ihre Salze, spezielle Formaldehyd-Donoren, spezielle Isothiazolinone, spezielle Parabenester und ihre Salze und spezielle Pyridone und ihre Salze sowie ihre Verwendung zum Konservieren von kosmetischen, dermatologischen oder pharmazeutischen Produkten.

In der DE 10 2009 022 445 (Clariant) werden flüssige Zusammensetzungen enthaltend Sorbitanmonocaprylat und Alkohol und ihre Verwendung zum Konservieren von kosmetischen, dermatologischen oder pharmazeutischen Produkten offenbart.

WO 2010/108738 A2 (Evonik) beschreibt Formulierungen zur Reinigung und Pflege von menschlichen oder

tierischen Körperteilen enthaltend Sorbitancarbonsäureester, wobei sich der Carbonsäureanteil des Sorbitancarbonsäureesters ableitet von einer Carbonsäure enthaltend 6 bis 10 Kohlenstoffatome und die Sorbitancarbonsäureester eine Hydroxylzahl (OH-Zahl) von größer 350 aufweisen sowie die Verwendung der genannten Sorbitancarbonsäureester als Viskositätsregler, Pflegewirkstoff, Schaumbooster oder Solubilisator in reinigenden oder pflegenden Formulierungen.

JP 8173787 (A) (Lion) beschreibt eine Zusammensetzung enthaltend einen oberflächenaktiven Stoff enthaltend einen Fettsäureester von dehydratisiertem Sorbitol und die Verwendung als Öl-in-Wasser-Emulgator und als Reinigungsbasis. Die Zusammensetzungen können Mono- oder Diester von Capryl- und/oder Caprinsäure mit einem Polyol ausgewählt aus der Gruppe bestehend aus 1,5-Sorbitan, 1,4-Sorbitan und Isosorbid enthalten. Isosorbidcaprylate/caprate und Sorbitancaprylate/caprate können z. B. nach dem Fachmann geläufigen Methoden hergestellt werden. Beispielsweise können diese Verbindungen durch Veresterung von Sorbitol oder Isosorbid nach üblichen und dem Fachmann bekannten Methoden hergestellt werden, wobei sowohl Sorbitol und Isosorbid selbst als auch die zur Veresterung verwendeten Säurekomponenten wiederum käuflich erwerbbar sind.

Durch die erfindungsgemäße Verwendung wird die Wirkung der Antiperspirantien und Deodorantien weiterhin bevorzugt hinsichtlich der Dauer der Verringerung von Körpergeruch verbessert.

Vorzugsweise enthält die Zusammensetzung A neben dem einen oder den mehreren Isosorbidcaprylaten/capraten eine oder mehrere weitere Verbindungen ausgewählt aus der Gruppe bestehend aus Caprylsäure, Caprinsäure, Sorbitol, Sorbitolcaprylaten, Sorbitolcapraten, Sorbitan und Isosorbid und gegebenenfalls ein oder mehrere Sorbitancaprylate/caprate.

Bei den Sorbitolcaprylaten und Sorbitolcapraten kann es sich um die entsprechenden Mono-, Di-, Tri-, Tetra-, Penta- und Hexaester von Sorbitol handeln oder um beliebige Mischungen aus diesen Substanzen.

Weiterhin bevorzugt ist die Gesamtmenge an dem einen oder den mehreren Isosorbidcaprylaten/capraten und dem gegebenenfalls einen oder mehreren zusätzlich in der Zusammensetzung A enthaltenen Sorbitancaprylaten/capraten in der Zusammensetzung A mindestens 50,0 Gew.-%, vorzugsweise mindestens 60,0 Gew.-%, besonders bevorzugt mindestens 70,0 Gew.-% und insbesondere bevorzugt mindestens 75,0 Gew.-%, bezogen auf das Gesamtgewicht der Zusammensetzung A.

Weiterhin bevorzugt enthält die Zusammensetzung A Isosorbidmonocaprylat/caprat.

Weiterhin bevorzugt enthält die Zusammensetzung A neben Isosorbidmonocaprylat/ caprat zusätzlich Sorbitanmonocaprylat/caprat.

Besonders bevorzugt ist die Gesamtmenge an Isosorbidmonocaprylat/caprat und dem gegebenenfalls zusätzlich in der Zusammensetzung A enthaltenen Sorbitanmonocaprylat/caprat in der Zusammensetzung A mindestens 30,0 Gew.-%, vorzugsweise mindestens 35,0 Gew.-%, besonders bevorzugt mindestens 40,0 Gew.-% und insbesondere bevorzugt mindestens 45 Gew.-%, bezogen auf das Gesamtgewicht der Zusammensetzung A. Hierbei kann die Gesamtmenge an Isosorbidmonocaprylat/ caprat und dem gegebenenfalls zusätzlich in der Zusammensetzung A enthaltenen Sorbitanmonocaprylat/caprat in der Zusammensetzung A bis 100 Gew.-% betragen. In einer bevorzugten Ausführungsform der Erfindung beträgt die Gesamtmenge an Isosorbidmonocaprylat/caprat und dem gegebenenfalls zusätzlich in der Zusammensetzung A enthaltenen Sorbitanmonocaprylat/caprat in der Zusammensetzung A jedoch nur bis zu 90,0 Gew.-%, vorzugsweise bis zu 80,0 Gew.-% und besonders bevorzugt bis zu 70,0 Gew.-%.

Weiterhin besonders bevorzugt enthält die Zusammensetzung A neben Isosorbidmonocaprylat/caprat zusätzlich Sorbitanmonocaprylat/caprat und ist das Gewichtsverhältnis von Sorbitanmonocaprylat/caprat zu Isosorbidmonocaprylat/caprat in der Zusammensetzung A von 20 : 1 bis 1 : 100, vorzugsweise von 10 : 1 bis 1 : 10, besonders bevorzugt von 6 : 1 bis 1 : 6, insbesondere bevorzugt von 3 : 1 bis 1 : 5 und außerordentlich bevorzugt von 1 : 1 bis 1 : 4.

In einer bevorzugten Ausführungsform der Erfindung enthalten die Zusammensetzungen A entweder keine Caprylsäure und Caprinsäure oder bis zu 1,0 Gew.-% Caprylsäure und Caprinsäure.

In einer weiteren bevorzugten Ausführungsform der Erfindung ist die OH-Zahl der in der Zusammensetzung A enthaltenen Mischung aus dem einen oder den mehreren Isosorbidcaprylaten/capraten, dem gegebenenfalls einen oder mehreren zusätzlich darin enthaltenen Sorbitancaprylaten/capraten und der gegebenenfalls einen oder mehreren zusätzlich darin enthaltenen Verbindungen ausgewählt aus der Gruppe bestehend aus Caprylsäure, Caprinsäure, Sorbitol, Sorbitolcaprylaten, Sorbitolcapraten, Sorbitan und Isosorbid oder der aus dieser Mischung bestehenden Zusammensetzung A kleiner oder gleich 460, vorzugsweise kleiner oder gleich 390, besonders bevorzugt kleiner oder gleich 340, insbesondere bevorzugt kleiner oder gleich 260 und außerordentlich bevorzugt kleiner oder gleich 225.

In einer weiteren bevorzugten Ausführungsform der Erfindung enthalten die Zusammensetzungen A keine Verbindungen ausgewählt aus Sorbitol, Sorbitolcaprylaten und Sorbitolcapraten.

In einer weiteren bevorzugten Ausführungsform der Erfindung bestehen die Zusammensetzungen A aus dem einen oder den mehreren Isosorbidcaprylaten/capraten, gegebenenfalls zusätzlich dem einen oder den mehreren Sorbitancaprylaten/capraten und gegebenenfalls zusätzlich der einen oder den mehreren Verbindungen ausgewählt aus der Gruppe bestehend aus Caprylsäure, Caprinsäure, Sorbitol, Sorbitolcaprylaten, Sorbitolcapraten, Sorbitan und Isosorbid.

Sofern die Zusammensetzungen A eine oder mehrere Verbindungen ausgewählt aus Sorbitol, Sorbitolcaprylaten und Sorbitolcapraten enthalten, sind diese Verbindungen gemeinsam vorzugsweise in einer Menge kleiner oder gleich 5,0 Gew.-%, besonders bevorzugt in einer Menge kleiner oder gleich 3,0 Gew.-%, insbesondere bevorzugt in einer Menge kleiner oder gleich 1,0 Gew.-% und außerordentlich bevorzugt in einer Menge kleiner oder gleich 0,5 Gew.-% in den Zusammensetzungen A enthalten, wobei die Angaben in Gew.-% jeweils auf das Gesamtgewicht der fertigen Zusammensetzungen A bezogen sind.

Unter der Hydroxyl- oder OH-Zahl einer Substanz wird diejenige Menge KOH in mg verstanden, die der bei der Acetylierung von 1 g Substanz gebundenen Menge an Essigsäure äquivalent ist.

Geeignete Bestimmungsmethoden zur Ermittlung der OH-Zahl sind z. B. DGF C-V 17 a (53), Ph. Eur. 2.5.3 Method A und DIN 53240.

Im Rahmen der vorliegenden Erfindung werden die OH-Zahlen in Anlehnung an DIN 53240-2 bestimmt. Hierbei wird wie folgt vorgegangen: Es wird 1 g auf 0,1 mg genau von der homogenisierten zu messenden Probe eingewogen. 20,00 ml Acetylierungsgemisch (Acetylierungsgemisch: in 1 Liter Pyridin werden 50 ml Essigsäureanhydrid eingerührt) werden zugeben. Die Probe wird vollständig im Acetylierungsgemisch gelöst, gegebenenfalls unter Rühren und Erwärmen. 5 ml Katalysatorlösung (Katalysatorlösung: 2 g 4-Dimethylaminopyridin werden in 100 ml Pyridin gelöst) werden zugeben. Das Reaktionsgefäß wird verschlossen und 10 Minuten in das auf 55 °C vorgeheizte Wasserbad gestellt und dabei durchmischt. Die Reaktionslösung wird danach mit 10 ml vollentsalztem Wasser versetzt, das Reaktionsgefäß erneut verschlossen und nochmals im Schüttelwasserbad 10 Minuten abreagieren gelassen. Die Probe wird auf Raumtemperatur (25 °C) abgekühlt. Anschließend werden 50 ml 2-Propanol und 2 Tropfen Phenolphthalein zugeben. Diese Lösung wird mit Natronlauge (Natronlauge c = 0,5 mol/l) titriert (Va). Unter den gleichen Bedingungen, jedoch ohne Probeneinwaage, wird der Wirkwert des Acetylierungsgemisches bestimmt (Vb).

Aus dem Verbrauch der Wirkwertbestimmung und der Titration der Probe wird die OH-Zahl (OHZ) nach folgender Formel berechnet:

$$OHZ = \frac{(Vb - Va) \cdot c \cdot t \cdot M}{E}$$

| | |
|---|---|
| OHZ = | Hydroxylzahl in mg KOH/g Substanz |
| Va = | Verbrauch an Natronlauge in ml bei der Titration der Probe |
| Vb = | Verbrauch an Natronlauge in ml bei der Titration des Wirkwertes |
| c = | Stoffmengenkonzentration der Natronlauge in mol/l |
| t = | Titer der Natronlauge |
| M = | Molare Masse von KOH = 56,11 g/mol |
| E = | Probeneinwaage in g |

(Vb - Va) ist diejenige Menge der verwendeten Natronlauge in ml, die der bei der oben beschriebenen Acetylierung der zu messenden Probe gebundenen Menge an Essigsäure äquivalent ist.

Die Menge der Zusammensetzung A in dem Antiperspirant oder Deodorant ist vorzugsweise von 0,1 bis 20,0 Gew.-%, besonders bevorzugt von 0,5 bis 15,0 Gew.-%, insbesondere bevorzugt von 2,0 bis 13,0 Gew.-% und außerordentlich bevorzugt von 3,0 bis 10,0 Gew.-%, bezogen auf das Gesamtgewicht des Antiperspirants oder Deodorants.

Vorzugsweise enthalten die Antiperspirantien und Deodorantien keine Aluminiumhaltigen Verbindungen.

In einer besonders bevorzugten Ausführungsform der Erfindung enthalten die Antiperspirantien und Deodorantien keine weiteren gegen Körpergeruch aktive Substanzen. Unter weiteren gegen Körpergeruch aktive Substanzen werden im Rahmen der vorliegenden Erfindung insbesondere Verbindungen verstanden, die zu Caprylsäure, Caprinsäure, Sorbitol, Sorbitolcaprylaten, Sorbitolcapraten, Sorbitan, Sorbitancaprylaten/capraten, Isosorbid und Isosorbidcaprylaten/capraten verschieden sind.

In einer weiteren besonders bevorzugten Ausführungsform der Erfindung enthalten die Antiperspirantien und Deodorantien eine oder mehrere weitere gegen Körpergeruch aktive Substanzen.

Vorzugsweise ist die eine oder sind die mehreren weiteren gegen Körpergeruch aktiven Substanzen ausgewählt aus der Gruppe bestehend aus Acorus Gramineus Root/Stem/Luffa Cylindrica Fruit/Camellia Sinensis Leaf Extract, Adipic Acid/Neopentyl Glycol Crosspolymer, Alpinia Uraiensis Stalk/Leaf Water, Amber Powder, Ammonium Phenolsulfonate, Ammonium Silver Zeolite, Ammonium Silver Zinc Aluminum Silicate, Benzalkonium Bromide, Benzalkonium Cetyl Phosphate, Benzalkonium Chloride, Benzalkonium Saccharinate, Benzethonium Chloride, Boesenbergia Pandurata Rhizome Extract, Bromochlorophene, Bursera Graveolens Fruit Oil, Butyl Acrylate/Ethyltrimonium Chloride Methacrylate/Styrene Copolymer, t-Butyl Methylphenoxy Phenol, Butyloctanoic Acid, Calcium Magnesium Silicate, Callicarpa Macrophylla Flower Extract, Candida Bombicola/Glucose/Methyl Rapeseedate Ferment, Capramidoethyl Capramidopropyldimonium Methosulfate, Capryloyl Gold of Pleasure Amino Acids, Caprylyl Glycol, Castanea Crenata (Chestnut) Pellicle Extract, Cetylpyridinium Chloride, Chitosan, Chlorophyllin-Copper Complex, Chlorothymol, Chloroxylenol, Citrus Reticulata (Tangerine) Peel Oil, Cloflucarban, Coix Lacryma-Jobi Ma-Yuen Seed/Pueraria Lobata Root/Ganoderma Lucidum (Mushroom) Extract, Colloidal Platinum, Curcuma Heyneana Root Powder, Cyclopentadecanone, Dequalinium Chloride, Dichlorophene, Dichloro-m-Xylenol, Dimethicone/PEG-15 Crosspolymer, Dimethylbicycloheptyl Ethanone, Dipotassium Capryloyl Glutamate, Disodium Capryloyl Glutamate, Disodium Dihydroxyethyl Sulfosuccinylundecylenate, Domiphen Bromide, Ethylhexylglycerin, Fermented Vegetable, Ferric Chloride, Geranic Acid, Glyceryl Caprylate, Glyceryl Caprylate/Caprate, Hexachlorophene, Hexanediol/PEG-2 Cocomonium Chloride/TDI Copolymer, Humulus Lupulus (Hops) Cone Extract, Hydrolyzed Cellulose, Hydrolyzed Sasa Veitchii Extract, Ketoglutaric Acid, Hypericum Perforatum Flower/Twig Extract, Lauryl Isoquinolinium Bromide, Laurylpyridinium Chloride, Macelignan, Mentha Aquatica Water, Methylbenzethonium Chloride, 2-Methyl 5-Cyclohexylpentanol, Methyl Phenylbutanol, Methyl Undecylenate, Michelia Champaca Flower Oil, Micrococcus/Hydrolyzed Nonfat Milk Ferment, Octadecenedioic Acid, Octanediol, Octenidine HCl, Oligopeptide-10, Oxidized Beta-Glucan, Pandanus Amaryllifolius Leaf Extract, Panduratin A, Pelargonium Graveolens Water, Phenol, Phyllostachys Edulis Stem Extract, Piper Betle Leaf Oil, Piroctonol, Piroctone Olamine, Polyaminopropyl Biguanide Stearate, Potassium Capryloyl Glutamate, Rapeseed Sophorolipids, Rosmarinus Officinalis (Rosemary) Flower Extract, Saccharomyces/Persimmon Fruit Juice Ferment Extract, Saccharomyces/Rhodobacter/Lactobacillus/Leuconostoc/Streptomyces Griseus/Aspergillus/Bacillus Ferment Filtrate, Sasa Senanensis Leaf Extract, Sasa Senanensis Leaf Powder, Scallop Shell Powder, Shikimic Acid, Silver Citrate, Silver Chloride (and) Titanium Dioxide, Silver Chloride (and) Titanium Dioxide (and) Diethylhexyl Sodium Sulfosuccinate (and) Propylene Glycol, Silver Copper Zeolite, Silver Lactate, Sodium Bicarbonate, Sodium Capryloyl Glutamate, Sodium Phenolsulfonate, Stemmacantha Carthamoides Root Extract, Totarol, Triclocarban, Triclosan, Tricyclodecenyl Propionate, Triethylcitrate, Urginea Maritima Tuber Extract, Zeolite, Zinc Dimethicone PEG-8 Succinate, Zinc Lactate, Zinc Phenolsulfonate, Zinc Ricinoleate, Zinc Silicate und Zirconium Powder.

Besonders bevorzugt ist die eine oder sind die mehreren weiteren gegen Körpergeruch aktiven Substanzen ausgewählt aus der Gruppe bestehend aus Caprylyl Glycol, Chitosan, Ethylhexylglycerin, Glyceryl Caprylate, Glyceryl Caprylate/Caprate, Octanediol, Piroctonol, Piroctone Olamine, Silver Citrate, Silver Chloride (and) Titanium Dioxide, Silver Chloride (and) Titanium Dioxide (and) Diethylhexyl Sodium Sulfosuccinate (and) Propylene Glycol, Silver Lactate, Triclosan, Triethylcitrate und Zinc Ricinoleate.

Insbesondere bevorzugt ist die eine oder sind die mehreren weiteren gegen Körpergeruch aktiven Substanzen ausgewählt aus der Gruppe bestehend aus Zinc Ricinoleate, Silver Chloride (and) Titanium Dioxide (and) Diethylhexyl Sodium Sulfosuccinate (and) Propylene Glycol, Piroctonol, Piroctone Olamine, Chitosan, Octandiol, Ethylhexylglycerin, Caprylyl Glycol, Glyceryl Caprylate, Glyceryl Caprylate/Caprate, Silber Citrate, Silber Lactate, Triclosan und Triethylcitrat.

Außerordentlich bevorzugt ist die eine oder sind die mehreren weiteren gegen Körpergeruch aktiven Substanzen ausgewählt aus der Gruppe bestehend aus Zinc Ricinoleate, Silver Chloride (and) Titanium Dioxide (and) Diethylhexyl Sodium Sulfosuccinate (and) Propylene Glycol (z. B. JM ActiCare®), Piroctonol, Chitosan, Octandiol und Piroctone Olamine.

Ganz besonders bevorzugt ist die eine oder sind die mehreren weiteren gegen Körpergeruch aktiven Substanzen ausgewählt aus der Gruppe bestehend aus

a.) Mischungen enthaltend Silberchlorid, Titandioxid, Diethylhexyl Natrium Sulfosuccinat und Propylenglykol (z. B. JM ActiCare®) und

b.) Chitosan, vorzugsweise mit mittleren molaren Massen von 10 000 bis 100 000 g/mol (z. B. Zenvivo® Protect oder Zenvivo® Aqua).

Die Antiperspirantien oder Deodorantien liegen vorzugsweise in Form von Sticks, Roll-ons, Fluids, Gelen, Sprays, Lotions oder Cremes vor.

Die Antiperspirantien oder Deodorantien sind vorzugsweise auf wässriger oder wässrig-alkoholischer Basis aufgebaut oder liegen als Emulsionen oder Dispersionen vor. Besonders bevorzugt liegen sie als Emulsionen vor und insbesondere bevorzugt liegen sie als Öl-in-Wasser Emulsionen vor.

Die Antiperspirantien oder Deodorantien können als weitere Hilfs- und Zusatzstoffe alle üblicherweise für diese Anwendung üblicherweise verwendeten Substanzen enthalten, beispielsweise Öle, Wachse, Emulgatoren, Co-Emulgatoren, Dispergatoren, Tenside, Entschäumer, Solubilisatoren, Elektrolyte, Hydroxysäuren, Stabilisatoren, Polymere, Filmbildner, Verdicker, Gelierungsmittel, Überfettungsmittel, Rückfetter, weitere antimikrobielle Wirkstoffe, biogene Wirkstoffe, Adstringentien, Aktivstoffe, Sonnenschutzfilter, Antioxidantien, Oxidantien, Feuchthaltemittel, Lösungsmittel, Farbmittel, Pigmente, Perlglanzmittel, Duftstoffe, Trübungsmittel und/oder Silikone.

Die Antiperspirantien oder Deodorantien besitzen pH-Werte von vorzugsweise 2 bis 11, besonders bevorzugt von 4,5 bis 8,5 und insbesondere bevorzugt von 5,0 bis 6,5.

Die nachfolgenden Beispiele sollen die Erfindung näher erläutern, ohne sie jedoch darauf zu beschränken.

1) Bestimmung der Verringerung des Körpergeruchs

Zur Bestimmung der Verringerung des Körpergeruchs wurden folgende Basisformulierungen für Roll-On-Deodorantien verwendet.

Basisformulierung I:

| Phase | Inhaltsstoff | Gew.-% |
|---|---|---|
| A | Wasser | ad 100 |
| B | Talkum (Luzenac® 00) | 9,0 |
| C | Ammonium Acryloyldimethyltaurate/Beheneth-25 Methacrylate Crosspolymer (Aristoflex® HMB)<br>Xanthan Gum | 0,6<br>0,1 |
| D | Squalane | 9,0 |
| E | Konservierungsmittel | q.s. |

Herstellung:

I Gebe B zu A unter Rühren
II Gebe C zu I und rühre bis die Lösung homogen ist
III Gebe D zu II
IV Gebe E zu III

Basisformulierung II:

| Phase | Inhaltsstoff | Gew.-% |
|---|---|---|
| A | Wasser | ad 100 |
| B | PEG-150 Distearate (Rewopal® PEG 6000 DS) | 1,0 |
| C | Ceteareth-25 (Genapol® T 250)<br>Butylene Glycol<br>Dicaprylyl Ether (Cetiol® OE)<br>Glyceryl Isostearate | 5,0<br>3,0<br>1,0<br>2,0 |
| D | Wasser<br>Milchsäure | 15,0<br>q.s. |
| E | Konservierungsmittel | q.s. |

Herstellung:

I Gebe bei 80 °C B zu A unter Rühren
II Löse C bei 80 °C und gebe I zu C unter Rühren
III Gebe D zu II
IV Gebe E zu III

In die Basisformulierungen wurden verschiedene gegen Körpergeruch aktive Substanzen eingearbeitet, beispielsweise als weiterer Bestandteil von Komponente D. Es handelte sich um die folgenden Substanzen:
A) Sorbitan-/Isosorbidcaprylat X1 folgender Zusammensetzung:

| Substanz | Gew.-% |
|---|---|
| Isosorbidmonocaprylat | 37,2 |
| Isosorbiddicaprylat | 15,1 |
| Sorbitanmonocaprylat | 11,6 |
| Sorbitandicaprylat | 9,1 |
| Sorbitantricaprylat | 4,2 |
| Sorbitantetracaprylat | 0,5 |
| Isosorbid | 15,9 |
| Sorbitan | 5,5 |
| Sorbitol | 0,1 |
| Caprylsäure | 0,8 |

B) Zinc Ricinoleate
C) JM ActiCare® folgender Zusammensetzung:

| Substanz | Gew.-% |
|---|---|
| Silberchlorid | 2 |
| Titandioxid | 8 |
| Diethylhexyl Natrium Sulfosuccinat | max. 20 |
| Propylenglykol | max. 5 |
| Wasser | min. 65 |

D) Piroctonol

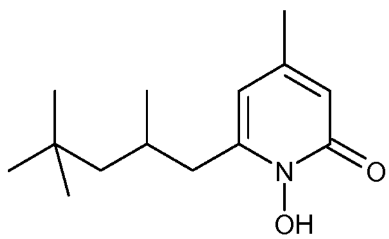

E) Zenvivo® Aqua

| Substanz | Gew.-% |
|---|---|
| Poly-(1-4)-2-Amino-2-deoxy-β-D-Glucan (Chitosan) mit mittlerer molarer Masse von 90.000 g/mol | min. 80 |
| β-D-Glucan | max. 10 |
| Wasser | max. 10 |

F) Zenvivo® Protect

| Substanz | Gew.-% |
|---|---|
| Poly-(1-4)-2-Amino-2-deoxy-β-D-Glucan (Chitosan) mit mittlerer molarer Masse von 15.000 g/mol | min. 75 |
| β-D-Glucan | max. 15 |
| Wasser | max. 10 |

Zur Bestimmung der Verringerung des Körpergeruchs wurde die deodorierende Wirkung im 48-Stunden-Sniff-Test durchgeführt, bei dem wie folgt vorgegangen wird:
20 Probanden werden nach den folgenden Kriterien ausgewählt:

- weiblich und männlich
- mind. 18 Jahre
- Nichtraucher
- klinisch gesund
- gut wahrnehmbarer axillärer Körpergeruch unter beiden Achseln

Die beiden Axillen der Probanden werden über mehrere Tage vorkonditioniert, d. h. es werden keinerlei Antiperspirantien und Deodorantien benutzt und eine pH-hautneutrale, unparfürmierte Flüssigseife verwendet.
An den 20 vorbehandelten Probanden wird in der linken oder rechten Axilla nach einmaliger Applikation von 250 - 300 mg der Basisformulierung enthaltend aktive Substanzen gegen Körpergeruch in der Komponente D von drei olfaktorischen Experten der axilläre Körpergeruch und Produktduft nach Intensität vor und nach Behandlung über insgesamt 48 Stunden bewertet.
Die entgegengesetzte Axilla wird nicht behandelt und dient als Standard zur Bestimmung der relativen Intensitätsabnahme des axillären Körpergeruches im Vergleich zur Axilla, die mit der Basisformulierung enthaltend aktive Substanzen gegen Körpergeruch in der Komponente D behandelt wurde.
Neben dem Ausgangswert unmittelbar vor der einmaligen Applikation (nach 0 Stunden) und dem Endwert 48 Stunden nach der einmaligen Applikation wird die Intensität des axillären Körpergeruches und Produktduftes auch nach 24 Stunden bewertet.
Die Intensität vom Körpergeruch bzw. Parfümduft wurde von den drei olfaktorischen Experten nach folgender Skala bewertet:

1 = nicht wahrnehmbar
2 = schwach wahrnehmbar
3 = wahrnehmbar
4 = stark wahrnehmbar
5 = sehr stark wahrnehmbar

Durch Vergleich der Intensitäten des Körpergeruchs (bzw. Parfümdufts) von der behandelten Axilla und der unbehandelten Axilla ergibt sich aus der untenstehenden Formel die relative Intensitätsabnahme des axillären Körpergeruches durch einmalige Applikation von 250-300 mg der Basisformulierung enthaltend aktive Substanzen gegen Körpergeruch in der Komponente D.

$$\textit{Intenstätsabnahme Körpergeruch} = \frac{\textit{(Geruch unbehandelte Axilla - Geruch behandelte Axilla)}}{\text{Geruch unbehandelte Axilla}} \cdot 100\ \%$$

2) Ergebnisse aus der Bestimmung zur Verringerung des Körpergeruchs

Es wurden folgende Ergebnisse erhalten:
Der Körpergeruch der unbehandelten Axilla blieb über den Studienzeitraum hinweg konstant.
Ein Parfümduft bzw. Eigengeruch des Prüfgegenstandes war nicht wahrnehmbar. Weitere Ergebnisse sind in den Tabellen A1 - A6 dargestellt.

Tabelle A1 Ergebnisse zu Versuchen zur Verringerung des Körpergeruchs unter Verwendung von Sorbitan-/ Isosorbidcaprylat X1 nach 24 und 48 Stunden

| zu Basisformulierung zugegebene Zusammensetzung | Basisformulierung | Ergebnis nach 24 Stunden [%] | Ergebnis nach 48 Stunden [%] |
|---|---|---|---|
| keine | I | 0 | 0 |
| 7,6 Gew.-% Sorbitan-/Isosorbidcaprylat X1 | I | 23 | 21 |
| keine | II | 0 | 0 |
| 1,0 Gew.-% Sorbitan-/Isosorbidcaprylat X1 | II | 10 | 9 |
| 4,0 Gew.-% Sorbitan-/Isosorbidcaprylat X1 | II | 21 | 20 |

An den Ergebnissen der Tabelle A1 erkennt man, dass die Inhaltsstoffe der Basisformulierung den Körpergeruch nicht verringern. Der Zusatz von bis zu 7,6 Gew.-% Sorbitan-/Isosorbidcaprylat X1 verringert die Intensität des Körpergeruches über den untersuchten Zeitraum von 48 Stunden nach Anwendung. Hierbei fällt auf, dass die Verringerung der Intensität des Körpergeruches überraschenderweise 48 Stunden nach Anwendung nur geringfügig unterhalb des Wertes 24 Stunden nach Anwendung liegt.

Tabelle A2 Ergebnisse zu Versuchen zur Verringerung des Körpergeruchs unter Verwendung von Sorbitan-/ Isosorbidcaprylat X1, Octandiol, Zinc Ricinoleate und Kombination nach 24 und 48 Stunden

| zu Basisformulierung I zugegebene Zusammensetzung | Ergebnis nach 24 Stunden [%] | Ergebnis nach 48 Stunden [%] |
|---|---|---|
| 7,6 Gew.-% Octandiol | 18 | 13 |
| 7,6 Gew.-% Sorbitan-/Isosorbidcaprylat X1 | 23 | 21 |
| 7,6 Gew.-% Octandiol und<br>1,8 Gew.-% Zinc Ricinoleate | 30 | 13 |
| 7,6 Gew.-% Sorbitan-/Isosorbidcaprylat X1 und<br>1,8 Gew.-% Zinc Ricinoleate | 28 | 23 |

An den Ergebnissen der Tabelle A2 erkennt man, dass sowohl Sorbitan-/ Isosorbidcaprylat X1 als auch Octandiol die Wirkung gegen Körpergeruch sowohl nach 24 als auch nach 48 Stunden verbessern. Hierbei führt das Sorbitan-/Isosorbidcaprylat X1 zu besseren Ergebnissen, insbesondere nach 48 Stunden.

Aus dem Vergleich der Ergebnisse für die Mischung aus Octandiol und Zinc Ricinoleate einerseits und für die Mischung aus Sorbitan-/Isosorbidcaprylat X1 und Zinc Ricinoleate andererseits erkennt man, dass die Wirkung gegen Körpergeruch nach 24 Stunden für beide Mischungen vergleichbar ist, aber die Mischung mit Sorbitan-/Isosorbidcaprylat X1 nach 48 Stunden zu einer signifikant besseren Wirkung gegen Körpergeruch führt.

Tabelle A3 Ergebnisse zu Versuchen zur Verringerung des Körpergeruchs unter Verwendung von Sorbitan-/ Isosorbidcaprylat X1 und JM ActiCare® nach 24 und 48 Stunden

| zu Basisformulierung I zugegebene Zusammensetzung | Ergebnis nach 24 Stunden [%] | Ergebnis nach 48 Stunden [%] |
|---|---|---|
| 0,2 Gew.-% JM ActiCare® | 21 | 21 |
| 0,2 Gew.-% JM ActiCare® und<br>7,6 Gew.-% Sorbitan-/Isosorbidcaprylat X1 | 30 | 25 |

An den Ergebnissen der Tabelle A3 erkennt man, dass Sorbitan-/Isosorbidcaprylat X1 in Antiperspirantien und Deodorantien die Wirkung von JM ActiCare® hinsichtlich der Stärke und Dauer der Verringerung von Körpergeruch

verbessert.

Tabelle A4 Ergebnisse zu Versuchen zur Verringerung des Körpergeruchs unter Verwendung von Sorbitan-/Isosorbidcaprylat X1 und Piroctonol nach 24 und 48 Stunden

| zu Basisformulierung I zugegebene Zusammensetzung | Ergebnis nach 24 Stunden [%] | Ergebnis nach 48 Stunden [%] |
|---|---|---|
| 0,1 Gew.-% Piroctonol, 5 Gew.-% Propylene Glycol[1] | 15 | 10 |
| 0,1 Gew.-% Piroctonol, 5 Gew.-% Propylene Glycol[1] und 7,6 Gew.-% Sorbitan-/Isosorbidcaprylat X1 | 30 | 21 |
| [1] 5 Gew.-% Propylene Glycol wird als Solubilisator für 0,1 Gew.-% Piroctonol zugesetzt. | | |

An den Ergebnissen der Tabelle A4 erkennt man, dass Sorbitan-/Isosorbidcaprylat X1 in Antiperspirantien und Deodorantien die Wirkung von Piroctonol hinsichtlich der Stärke und Dauer der Verringerung von Körpergeruch verbessert.

Tabelle A5 Ergebnisse zu Versuchen zur Verringerung des Körpergeruchs unter Verwendung von Sorbitan-/Isosorbidcaprylat X1 und Zenvivo® Aqua nach 24 und 48 Stunden

| zu Basisformulierung II zugegebene Zusammensetzung | Ergebnis nach 24 Stunden [%] | Ergebnis nach 48 Stunden [%] |
|---|---|---|
| 0,4 Gew.-% Zenvivo® Aqua | 32 | 26 |
| 1,0 Gew.-% Sorbitan-/Isosorbidcaprylat X1 + 0,4 Gew.-% Zenvivo® Aqua | 41 | 34 |

An den Ergebnissen der Tabelle A5 erkennt man, dass Sorbitan-/Isosorbidcaprylat X1 in Antiperspirantien und Deodorantien die Wirkung von Zenvivo® Aqua hinsichtlich der Stärke und Dauer der Verringerung von Körpergeruch verbessert.

Tabelle A6 Ergebnisse zu Versuchen zur Verringerung des Körpergeruchs unter Verwendung von Sorbitan-/Isosorbidcaprylat X1 und Zenvivo® Protect nach 24 und 48 Stunden

| zu Basisformulierung II zugegebene Zusammensetzung | Ergebnis nach 24 Stunden [%] | Ergebnis nach 48 Stunden [%] |
|---|---|---|
| 0,4 Gew.-% Zenvivo® Protect | 28 | 19 |
| 1,0 Gew.-% Sorbitan-/Isosorbidcaprylat X1 + 0,4 Gew.-% Zenvivo® Protect | 32 | 25 |

An den Ergebnissen der Tabelle A6 erkennt man, dass Sorbitan-/Isosorbidcaprylat X1 in Antiperspirantien und Deodorantien die Wirkung von Zenvivo® Protect hinsichtlich der Stärke und Dauer der Verringerung von Körpergeruch verbessert.

3) Formulierungsbeispiele

Die erfindungsgemäße Verwendung kann beispielsweise in folgenden Formulierungen stattfinden.

Formulierungsbeispiel 1: Roll On Deodorant

| Phase | Inhaltsstoff | Gew.-% |
|---|---|---|
| A | Hydroxyethyl Cellulose (Tylose® H 10000 G4) | 0,7 |
| B | Wasser | ad 100 |
| C | Piroctone Olamine (Octopirox®) | 0,2 |
| D | Ethanol | 30,0 |
| E | Sorbitan-/Isosorbidcaprylat X1<br>Propylene Glycol<br>PEG-40 Hydrogenated Castor Oil (Emulsogen® HCO 040) | 8,0<br>5,0<br>0,5 |
| F | Citric Acid (pH 5,0-5,5) | q.s. |

Herstellung:

I Gebe A zu B unter kontinuierlichem Rühren bis die Lösung homogen ist.
II Löse C in D und gebe die Komponenten aus E zu.
III Gebe II zu I.
IV Stelle den pH mit F ein.

Formulierungsbeispiel 2: Deodorant Stick

| Phase | Inhaltsstoff | Gew.-% |
|---|---|---|
| A | Piroctone Olamine (Octopirox®)<br>Sorbitan-/Isosorbidcaprylat X1<br>1,3-Butanediol<br>Propylene Glycol<br>Isosteareth-20 (Rewoderm® 66E)<br>Steareth-2 (Genapol® HS 020) | 0,1<br>4,0<br>30,0<br>27,0<br>4,0<br>1,0 |
| B | Polyglykol 1500 (PEG-32) | 5,0 |
| C | Wasser | ad 100 |
| D | Sodium Stearate | 6,0 |

Herstellung:

I Mische die Komponenten aus A, heize auf ca. 50 °C auf und homogenisiere unter Rühren.

II Löse B in C unter Rühren und heize auf ca. 50 °C auf.

III Mische I und II, gebe D zu und löse D unter Rühren und Heizen bis die Lösung klar ist.

IV Fülle III in Deo Stift Gehäuse ein und lasse die Formulierung abkühlen.

Formulierungsbeispiel 3: Deodorant Gel

| Phase | Inhaltsstoff | Gew.-% |
|---|---|---|
| A | PEG-40 Hydrogenated Castor Oil (Emulsogen® HCO 040) | 1,0 |
| | Ethanol | 25,0 |
| | Piroctone Olamine (Octopirox®) | 0,1 |
| | Sorbitan-/Isosorbidcaprylat X1 | 3,0 |
| B | Propylene Glycol | 20,0 |
| | Diisopropyl Adipate (Isoadipate® 660014) | 1,0 |
| | Wasser | ad 100 |
| C | Citric Acid | q.s. |
| D | Ammonium Acryloyldimethyltaurate/VP Copolymer (Aristoflex® AVC) | 1,3 |

Herstellung:

I Mische die Komponenten aus A bis die Lösung klar ist.
II Gebe B unter Rühren zu I.
III Stelle den pH von II mit C auf 5,5 - 6,0 ein.
IV Gebe D unter Rühren zu III.

Formulierungsbeispiel 4: Antiperspirant in Form eines Fußsprays

| Phase | Inhaltsstoff | Gew.-% |
|---|---|---|
| A | Piroctone Olamine (Octopirox®) | 0,3 |
| | Menthol | 0,1 |
| | Sorbitan-/Isosorbidcaprylat X1 | 2,0 |
| B | Ethanol | 55,0 |
| | Propylene Glycol | 5,0 |
| C | Allantoin | 0,1 |
| | Panthenol | 0,5 |
| | Wasser | ad 100 |
| D | Citric Acid | q.s. |

Herstellung:

I Löse A in B.
II Gebe erwärmtes C unter Rühren zu I.
III Stelle den pH von II mit D auf 5,5 - 6,0 ein.

Formulierungsbeispiel 5: Deodorant in Form einer Fußcreme

| Phase | Inhaltsstoff | Gew.-% |
|---|---|---|
| A | Piroctone Olamine (Octopirox®)<br>Propylene Glycol | 0,2<br>2,0 |
| B | Triceteareth-4 Phosphate (Hostaphat® KW 340 D)<br>Sorbitan-/Isosorbidcaprylat X1<br>Caprylyl Methicone (SilCare® Silicone 41M15)<br>PEG-4 Polyglyceryl-2 Stearate (Hostacerin® DGSB)<br>Cetearyl Alcohol<br>Caprylic/Capric Triglyceride (Velsan® CCT) | 2,5<br>5,0<br>1,0<br>1,5<br>2,0<br>4,0 |
| C | Ammonium Acryloyldimethyltaurate/VP Copolymer (Aristoflex® AVC) | 0,8 |
| D | Wasser | ad 100 |
| E | Propylene Glycol<br>Camphor<br>Menthol | 2,0<br>0,1<br>0,2 |
| F | Citric Acid | q.s. |

Herstellung:

I Mische die Komponenten von A und erwärme unter Rühren bis die Lösung klar ist.
II Gebe die Komponenten von B zu I und erwärme unter Rühren auf 60 °C.
III Gebe C zu II.
IV Erwärme D auf ca. 60 °C und gebe es unter Rühren zu III.
V Gebe die erwärmte Mischung der Komponenten aus E in IV zu.
VI Stelle den pH von V mit F auf 5,5 - 6,0 ein.

Bei den Formulierungsbeispielen 1 - 5 kann man statt Piroctone Olamine auch Zinc Ricinoleate, JM ActiCare®, Piroctonol, Chitosan (Zenvivo® Aqua, Zenvio® Protect), Octandiol, Ethylhexylglycerin, Caprylyl Glycol, Glyceryl Caprylate, Glyceryl Caprylate/Caprate, Silber Citrate, Silber Lactate, Triclosan und Triethylcitrat als zusätzliche gegen Körpergeruch aktive Substanz neben Sorbitan-/Isosorbidcaprylat X1 einarbeiten.

Bei den Formulierungsbeispielen 1 - 5 kann man statt Sorbitan-/Isosorbidcaprylat X1 auch Sorbitan-/Isosorbidcaprylat X2, Isosorbidcaprylat X3, Sorbitan-/Isosorbidcaprat X4, Sorbitan-/Isosorbidcaprat X5, Isosorbidcaprat X6, Sorbitan-/Isosorbidcaprylat/caprat X7, Sorbitan-/Isosorbidcaprylat/caprat X8 und Isosorbidcaprylat/caprat X9 einarbeiten.

G) Sorbitan-/Isosorbidcaprylat X2 folgender Zusammensetzung:

| Substanz | Gew.-% |
|---|---|
| Isosorbidmonocaprylat | 18,0 |
| Isosorbiddicaprylat | 5,0 |
| Sorbitanmonocaprylat | 22,1 |
| Sorbitandicaprylat | 21,0 |
| Sorbitantricaprylat | 8,6 |
| Sorbitantetracaprylat | 1,0 |
| Isosorbid | 15,8 |
| Sorbitan | 8,1 |

(fortgesetzt)

| Substanz | Gew.-% |
|---|---|
| Sorbitol | 0,1 |
| Caprylsäure | 0,3 |

H) Isosorbidcaprylat X3 folgender Zusammensetzung:

| Substanz | Gew.-% |
|---|---|
| Isosorbidmonocaprylat | 50,9 |
| Isosorbiddicaprylat | 30,6 |
| Isosorbid | 18,1 |
| Caprylsäure | 0,4 |

K) Sorbitan-/Isosorbidcaprat X4 folgender Zusammensetzung:

| Substanz | Gew.-% |
|---|---|
| Isosorbidmonocaprat | 34,2 |
| Isosorbiddicaprat | 13,1 |
| Sorbitanmonocaprat | 14,6 |
| Sorbitandicaprat | 10,3 |
| Sorbitantricaprat | 5,0 |
| Sorbitantetracaprat | 0,5 |
| Isosorbid | 15,9 |
| Sorbitan | 5,5 |
| Sorbitol | 0,1 |
| Caprinsäure | 0,8 |

L) Sorbitan-/Isosorbidcaprat X5 folgender Zusammensetzung:

| Substanz | Gew.-% |
|---|---|
| Isosorbidmonocaprat | 16,0 |
| Isosorbiddicaprat | 5,0 |
| Sorbitanmonocaprat | 24,1 |
| Sorbitandicaprat | 21,0 |
| Sorbitantricaprat | 8,6 |
| Sorbitantetracaprat | 1,0 |
| Isosorbid | 15,8 |
| Sorbitan | 8,1 |

(fortgesetzt)

| Substanz | Gew.-% |
|---|---|
| Sorbitol | 0,1 |
| Caprinsäure | 0,3 |

M) Isosorbidcaprat X6 folgender Zusammensetzung:

| Substanz | Gew.-% |
|---|---|
| Isosorbidmonocaprat | 53,4 |
| Isosorbiddicaprat | 28,6 |
| Isosorbid | 17,6 |
| Caprinsäure | 0,4 |

N) Sorbitan-/Isosorbidcaprylat/caprat X7 folgender Zusammensetzung:

| Substanz | Gew.-% |
|---|---|
| Isosorbidmonocaprylat | 18,9 |
| Isosorbidmonocaprat | 18,3 |
| Isosorbiddicaprylat/caprat [a)] | 15,1 |
| Sorbitanmonocaprylat | 6,0 |
| Sorbitanmonocaprat | 5,6 |
| Sorbitandicaprylat/caprat [a)] | 9,1 |
| Sorbitantricaprylat/caprat [a)] | 4,2 |
| Sorbitantetracaprylat/caprat [a)] | 0,5 |
| Isosorbid | 15,9 |
| Sorbitan | 5,5 |
| Sorbitol | 0,1 |
| Caprylsäure | 0,4 |
| Caprinsäure | 0,4 |
| [a)] Die für die Zusammensetzung von X7 angegebenen Begriffe wie "Isosorbiddicaprylat/caprat" bedeuten, dass auf Grund der mehreren Esterbindungen in einem Molekül sowohl reine Caprylate oder reine Caprate als auch Mischungen aus Caprylaten und Capraten vorliegen können. | |

P) Sorbitan-/Isosorbidcaprylat/caprat X8 folgender Zusammensetzung:

| Substanz | Gew.-% |
|---|---|
| Isosorbidmonocaprylat | 9,1 |
| Isosorbidmonocaprat | 8,9 |

(fortgesetzt)

| Substanz | Gew.-% |
|---|---|
| Isosorbiddicaprylat/caprat [a)] | 5,0 |
| Sorbitanmonocaprylat | 11,2 |
| Sorbitanmonocaprat | 10,9 |
| Sorbitandicaprylat/caprat [a)] | 21,0 |
| Sorbitantricaprylat/caprat [a)] | 8,6 |
| Sorbitantetracaprylat/caprat [a)] | 1,0 |
| Isosorbid | 15,8 |
| Sorbitan | 7,9 |
| Sorbitol | 0,1 |
| Caprylsäure | 0,3 |
| Caprinsäure | 0,2 |
| [a)] siehe Erklärung bei N) Sorbitan-/Isosorbidcaprylat/caprat X7 | |

R) Isosorbidcaprylat/caprat X9 folgender Zusammensetzung:

| Substanz | Gew.-% |
|---|---|
| Isosorbidmonocaprylat | 25,9 |
| Isosorbidmonocaprat | 25,0 |
| Isosorbiddicaprylat/caprat [a)] | 30,6 |
| Isosorbid | 18,1 |
| Caprylsäure | 0,2 |
| Caprinsäure | 0,2 |
| [a)] siehe Erklärung bei N) Sorbitan-/Isosorbidcaprylat/caprat X7 | |

## Patentansprüche

1. Verwendung einer Zusammensetzung enthaltend ein oder mehrere Isosorbidcaprylate/caprate (Zusammensetzung A) in Antiperspirantien und Deodorantien zur Verbesserung der Wirkung der Antiperspirantien und Deodorantien hinsichtlich der Dauer der Verringerung von Körpergeruch.

2. Verwendung nach Anspruch 1, **dadurch gekennzeichnet, dass** die Zusammensetzung A neben dem einen oder den mehreren Isosorbidcaprylaten/ capraten zusätzlich ein oder mehrere Sorbitancaprylate/caprate enthält.

3. Verwendung nach Anspruch 1 oder 2, **dadurch gekennzeichnet, dass** die Wirkung der Antiperspirantien und Deodorantien hinsichtlich der Stärke der Verringerung von Körpergeruch verbessert wird.

4. Verwendung nach einem oder mehreren der Ansprüche 1 bis 3, **dadurch gekennzeichnet, dass** die Zusammensetzung A neben dem einen oder den mehreren Isosorbidcaprylaten/capraten eine oder mehrere weitere Verbindungen ausgewählt aus der Gruppe bestehend aus Caprylsäure, Caprinsäure, Sorbitol, Sorbitolcaprylaten, Sorbitolcapraten, Sorbitan und Isosorbid und gegebenenfalls ein oder mehrere Sorbitancaprylate/caprate enthält.

5. Verwendung nach einem oder mehreren der Ansprüche 1 bis 4, **dadurch gekennzeichnet, dass** die Gesamtmenge

an dem einen oder den mehreren Isosorbidcaprylaten/capraten und dem gegebenenfalls einen oder mehreren zusätzlich in der Zusammensetzung A enthaltenen Sorbitancaprylaten/capraten in der Zusammensetzung A mindestens 50,0 Gew.-%, bezogen auf das Gesamtgewicht der Zusammensetzung A, ist.

**6.** Verwendung nach einem oder mehreren der Ansprüche 1 bis 5, **dadurch gekennzeichnet, dass** die Gesamtmenge an dem einen oder den mehreren Isosorbidcaprylaten/capraten und dem gegebenenfalls einen oder mehreren zusätzlich in der Zusammensetzung A enthaltenen Sorbitancaprylaten/capraten in der Zusammensetzung A mindestens 60,0 Gew.-%, besonders bevorzugt mindestens 70,0 Gew.-% und insbesondere bevorzugt mindestens 75,0 Gew.-%, bezogen auf das Gesamtgewicht der Zusammensetzung A, ist.

**7.** Verwendung nach einem oder mehreren der Ansprüche 1 bis 6, **dadurch gekennzeichnet, dass** die Zusammensetzung A Isosorbidmonocaprylat/caprat enthält.

**8.** Verwendung nach Anspruch 7, **dadurch gekennzeichnet, dass** die Zusammensetzung A neben Isosorbidmonocaprylat/caprat zusätzlich Sorbitanmonocaprylat/caprat enthält.

**9.** Verwendung nach Anspruch 7 oder 8, **dadurch gekennzeichnet, dass** die Gesamtmenge an Isosorbidmonocaprylat/caprat und dem gegebenenfalls zusätzlich in der Zusammensetzung A enthaltenen Sorbitanmonocaprylat/caprat in der Zusammensetzung A mindestens 30,0 Gew.-%, vorzugsweise mindestens 35,0 Gew.-%, besonders bevorzugt mindestens 40,0 Gew.-% und insbesondere bevorzugt mindestens 45 Gew.-%, bezogen auf das Gesamtgewicht der Zusammensetzung A, ist.

**10.** Verwendung nach Anspruch 8 oder 9, **dadurch gekennzeichnet, dass** die Zusammensetzung A neben Isosorbidmonocaprylat/caprat zusätzlich Sorbitanmonocaprylat/caprat enthält und das Gewichtsverhältnis von Sorbitanmonocaprylat/caprat zu Isosorbidmonocaprylat/caprat in der Zusammensetzung A von 20 : 1 bis 1 : 100, vorzugsweise von 10 : 1 bis 1 : 10, besonders bevorzugt von 6 : 1 bis 1 : 6, insbesondere bevorzugt von 3 : 1 bis 1 : 5 und außerordentlich bevorzugt von 1 : 1 bis 1 : 4 ist.

**11.** Verwendung nach einem oder mehreren der Ansprüche 1 bis 10, **dadurch gekennzeichnet, dass** die Menge der Zusammensetzung A in dem Antiperspirant oder Deodorant von 0,1 bis 20,0 Gew.-%, vorzugsweise von 0,5 bis 15,0 Gew.-%, besonders bevorzugt von 2,0 bis 13,0 Gew.-% und insbesondere bevorzugt von 3,0 bis 10,0 Gew.-% ist, bezogen auf das Gesamtgewicht des Antiperspirants oder Deodorants.

**12.** Verwendung nach einem oder mehreren der Ansprüche 1 bis 11, **dadurch gekennzeichnet, dass** die Antiperspirantien und Deodorantien keine aluminiumhaltigen Verbindungen enthalten.

**13.** Verwendung nach einem oder mehreren der Ansprüche 1 bis 12, **dadurch gekennzeichnet, dass** die Antiperspirantien und Deodorantien keine weiteren gegen Körpergeruch aktive Substanzen enthalten.

**14.** Verwendung nach einem oder mehreren der Ansprüche 1 bis 12, **dadurch gekennzeichnet, dass** die Antiperspirantien und Deodorantien eine oder mehrere weitere gegen Körpergeruch aktive Substanzen enthalten.

**15.** Verwendung nach Anspruch 14, **dadurch gekennzeichnet, dass** die eine oder die mehreren weiteren gegen Körpergeruch aktiven Substanzen ausgewählt sind aus der Gruppe bestehend aus Caprylyl Glycol, Chitosan, Ethylhexylglycerin, Glyceryl Caprylate, Glyceryl Caprylate/Caprate, Octanediol, Piroctonol, Piroctone Olamine, Silver Citrate, Silver Chloride (and) Titanium Dioxide, Silver Chloride (and) Titanium Dioxide (and) Diethylhexyl Sodium Sulfosuccinate (and) Propylene Glycol, Silver Lactate, Triclosan, Triethylcitrate und Zinc Ricinoleate.

## Claims

**1.** Use of a composition comprising one or more isosorbide caprylates/caprates (composition A) in antiperspirants and deodorants for improving the action of the antiperspirants and deodorants with regard to the duration of reducing body odour.

**2.** Use according to Claim 1, **characterized in that** the composition A additionally comprises one or more sorbitan caprylates/caprates besides the one or more isosorbide caprylates/caprates.

3. Use according to Claim 1 or 2, **characterized in that** the action of the antiperspirants and deodorants is improved as regards the extent of the reduction in body odour.

4. Use according to one or more of Claims 1 to 3, **characterized in that** the composition A comprises, besides the one or more isosorbide caprylates/caprates, one or more further compounds selected from the group consisting of caprylic acid, capric acid, sorbitol, sorbitol caprylates, sorbitol caprates, sorbitan and isosorbide and optionally one or more sorbitan caprylates/caprates.

5. Use according to one or more of Claims 1 to 4, **characterized in that** the total amount in the composition A of the one or more isosorbide caprylates/caprates and the optionally one or more sorbitan caprylates/caprates additionally present in the composition A is at least 50.0% by weight, based on the total weight of the composition A.

6. Use according to one or more of Claims 1 to 5, **characterized in that** the total amount in the composition A of the one or more isosorbide caprylates/caprates and the optionally one or more sorbitan caprylates/caprates additionally present in the composition A is at least 60.0% by weight, particularly preferably at least 70.0% by weight and particularly preferably at least 75.0% by weight, based on the total weight of the composition A.

7. Use according to one or more of Claims 1 to 6, **characterized in that** the composition A comprises isosorbide monocaprylate/caprate.

8. Use according to Claim 7, **characterized in that** the composition A additionally comprises sorbitan monocaprylate/caprate alongside isosorbide monocaprylate/caprate.

9. Use according to Claim 7 or 8, **characterized in that** the total amount in the composition A of isosorbide monocaprylate/caprate and the sorbitan monocaprylate/caprate optionally additionally present in the composition A is at least 30.0% by weight, preferably at least 35.0% by weight, particularly preferably at least 40.0% by weight and especially preferably at least 45% by weight, based on the total weight of the composition A.

10. Use according to Claim 8 or 9, **characterized in that** the composition A additionally comprises sorbitan monocaprylate/caprate alongside isosorbide monocaprylate/caprate and the weight ratio of sorbitan monocaprylate/caprate to isosorbide monocaprylate/caprate in the composition A is from 20:1 to 1:100, preferably from 10:1 to 1:10, particularly preferably from 6:1 to 1:6, especially preferably from 3:1 to 1:5 and extraordinarily preferably from 1:1 to 1:4.

11. Use according to one or more of Claims 1 to 10, **characterized in that** the amount of composition A in the antiperspirant or deodorant is from 0.1 to 20.0% by weight, preferably from 0.5 to 15.0% by weight, particularly preferably from 2.0 to 13.0% by weight and especially preferably from 3.0 to 10.0% by weight, based on the total weight of the antiperspirant or deodorant.

12. Use according to one or more of Claims 1 to 11, **characterized in that** the antiperspirants and deodorants comprise no aluminium-containing compounds.

13. Use according to one or more of Claims 1 to 12, **characterized in that** the antiperspirants and deodorants comprise no further substances active against body odour.

14. Use according to one or more of Claims 1 to 12, **characterized in that** the antiperspirants and deodorants comprise one or more further substances active against body odour.

15. Use according to Claim 14, **characterized in that** the one or more further substances active against body odour are selected from the group consisting of Caprylyl Glycol, Chitosan, Ethylhexylglycerin, Glyceryl Caprylate, Glyceryl Caprylate/Caprate, Octanediol, Piroctonol, Piroctone Olamine, Silver Citrate, Silver Chloride (and) Titanium Dioxide, Silver Chloride (and) Titanium Dioxide (and) Diethylhexyl Sodium Sulfosuccinate (and) Propylene Glycol, Silver Lactate, Triclosan, Triethyl citrate and Zinc Ricinoleate.

**Revendications**

1. Utilisation d'une composition contenant un ou plusieurs caprylates/caprates d'isosorbide (composition A) dans des antitranspirants et des déodorants pour l'amélioration de l'effet des antitranspirants et des déodorants en ce qui

concerne la durée de l'atténuation de l'odeur corporelle.

2. Utilisation selon la revendication 1, **caractérisée en ce que** la composition A contient de plus, outre le ou les caprylates/caprates d'isosorbide, un ou plusieurs caprylates/caprates de sorbitane.

3. Utilisation selon la revendication 1 ou 2, **caractérisée en ce que** l'effet des antitranspirants et des déodorants en ce qui concerne le pouvoir d'atténuation de l'odeur corporelle étant amélioré.

4. Utilisation selon l'une ou plusieurs des revendications 1 à 3, **caractérisée en ce que** la composition A contient, outre le ou les caprylates/caprates d'isosorbide, un ou plusieurs composés supplémentaires choisis dans le groupe constitué par l'acide caprylique, l'acide caprique, le sorbitol, des caprylates de sorbitol, des caprates de sorbitol, le sorbitane et l'isosorbide et éventuellement un ou plusieurs caprylates/caprates de sorbitane.

5. Utilisation selon l'une ou plusieurs des revendications 1 à 4, **caractérisée en ce que** la quantité totale du ou des caprylates/caprates d'isosorbide et du ou des caprylates/caprates de sorbitane éventuellement de plus contenus dans la composition A, dans la composition A est d'au moins 50,0 % en poids, par rapport au poids total de la composition A.

6. Utilisation selon l'une ou plusieurs des revendications 1 à 5, **caractérisée en ce que** la quantité totale du ou des caprylates/caprates d'isosorbide et du ou des caprylates/caprates de sorbitane éventuellement de plus contenus dans la composition A, dans la composition A est d'au moins 60,0 % en poids, particulièrement préférablement d'au moins 70,0 % en poids et en particulier préférablement d'au moins 75,0 % en poids, par rapport au poids total de la composition A.

7. Utilisation selon l'une ou plusieurs des revendications 1 à 6, **caractérisée en ce que** la composition A contient du monocaprylate/caprate d'isosorbide.

8. Utilisation selon la revendication 7, **caractérisée en ce que** la composition A contient de plus, outre le monocaprylate/caprate d'isosorbide, du monocaprylate/caprate de sorbitane.

9. Utilisation selon la revendication 7 ou 8, **caractérisée en ce que** la quantité totale de monocaprylate/caprate d'isosorbide et du monocaprylate/caprate de sorbitane éventuellement de plus contenu dans la composition A, dans la composition A est d'au moins 30,0 % en poids, de préférence d'au moins 35,0 % en poids, particulièrement préférablement d'au moins 40,0 % en poids et en particulier préférablement d'au moins 45 % en poids, par rapport au poids total de la composition A.

10. Utilisation selon la revendication 8 ou 9, **caractérisée en ce que** la composition A contient de plus, outre le monocaprylate/caprate d'isosorbide, du monocaprylate/caprate de sorbitane et le rapport pondéral de monocaprylate/caprate de sorbitane à monocaprylate/caprate d'isosorbide dans la composition A est de 20:1 à 1:100, de préférence de 10:1 à 1:10, particulièrement préférablement de 6:1 à 1:6, en particulier préférablement de 3:1 à 1:5 et extraordinairement préférablement de 1:1 à 1:4.

11. Utilisation selon l'une ou plusieurs des revendications 1 à 10, **caractérisée en ce que** la quantité de composition A dans l'antitranspirant ou le déodorant est de 0,1 à 20,0 % en poids, de préférence de 0,5 à 15,0 % en poids, particulièrement préférablement de 2,0 à 13,0 % en poids et en particulier préférablement de 3,0 à 10,0 % en poids, par rapport au poids total de l'antitranspirant ou du déodorant.

12. Utilisation selon l'une ou plusieurs des revendications 1 à 11, **caractérisée en ce que** les antitranspirants et les déodorants ne contiennent aucun composé contenant de l'aluminium.

13. Utilisation selon l'une ou plusieurs des revendications 1 à 12, **caractérisée en ce que** les antitranspirants et les déodorants ne contiennent aucune substance active supplémentaire contre l'odeur corporelle.

14. Utilisation selon l'une ou plusieurs des revendications 1 à 12, **caractérisée en ce que** les antitranspirants et les déodorants contiennent une ou plusieurs substances actives supplémentaires contre l'odeur corporelle.

15. Utilisation selon la revendication 14, **caractérisée en ce que** la ou les substances actives supplémentaires contre l'odeur corporelle sont choisies dans le groupe constitué par le caprylyle glycol, le chitosane, l'éthylhexylglycérine,

le caprylate de glycéryle, le caprylate/caprate de glycéryle, l'octanediol, le piroctonol, la piroctone-olamine, le citrate d'argent, le chlorure d'argent (et) les dioxydes de titane, le chlorure d'argent (et) les dioxydes de titane (et) le sulfosuccinate sodique de diéthylhexyle (et) le propylèneglycol, le lactate d'argent, le triclosan, le citrate de triéthyle et le ricinoléate de zinc.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102009022444
- DE 102009022445
- WO 2010108738 A2
- JP 8173787 A


### In der Beschreibung aufgeführte Nicht-Patentliteratur


- *Ph. Eur. 2.5.3 Method A und DIN 53240,* vol. 17 a (53